(19) **Europäisches Patentamt**
**European Patent Office**
**Office européen des brevets**

(11) **EP 4 645 321 A1**

(12) # EUROPEAN PATENT APPLICATION

(43) Date of publication:
**05.11.2025 Bulletin 2025/45**

(21) Application number: **24305710.6**

(22) Date of filing: **03.05.2024**

(51) International Patent Classification (IPC):
**G16B 40/30** (2019.01)      **G16B 45/00** (2019.01)

(52) Cooperative Patent Classification (CPC):
**G16B 45/00; G16B 40/30**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Metafora Biosystems**
**75014 Paris (FR)**

(72) Inventors:
• **PETIT, Vincent**
**75014 PARIS (FR)**
• **LABARTHE, Baptiste**
**75010 PARIS (FR)**
• **CZECHOWSKA-KUCIO, Kamila**
**6418 ROTHENTHURM (CH)**

(74) Representative: **Icosa**
**83 avenue Denfert-Rochereau**
**75014 Paris (FR)**

(54) **METHOD FOR CLUSTERING, VISUALIZATION AND INTERPRETATION OF SINGLE CELL DATA**

(57)    The present invention relates to a system (1) for clustering and visualization of single cell-type data, the system comprising: a visual display device (60); a user interface (50), and at least one computer processor (10) coupled to the visual display device (60) and the user interface (50) programmed to perform a computer-implemented method for clustering and visualization of single-cell type data associated with a plurality of biological objects.

**FIG. 1**

EP 4 645 321 A1

**Description**

**FIELD OF INVENTION**

The present invention relates to methods and systems for analyzing, notably trough visualization, single cell cytometry data associated with biological objects. More in detail the present invention relates to an interactive visualization method allowing the user to perform analysis and manipulation of biological objects organized in a clustering hierarchical structure, to better interpret data structure.

**BACKGROUND OF INVENTION**

Cytomics corresponds to the analysis of the cytome, which is the set of cellular constituents, including morphological, antigenic, and functional components, that allow defining or modeling the state and functioning of a cell at a given time t. Cytome analysis enables the description of the structural and functional heterogeneity of various cells within an organism.

Current approaches enable obtaining numerous insights into cellular and/or tissue responses to in vitro or in vivo exposure. They can be particularly useful for highlighting and identifying new biomarkers (for diagnosis, susceptibility, prognosis, exposure, or effect), generating new mechanistic knowledge (modes of action), or developing new tools for predictive efficacy or toxicology to aid in identifying new therapeutic targets or candidate drugs or vaccines.

The development of high-throughput methods, made possible in particular by the emergence of specialized technological platforms, have enabled the industrialization of data production and the simultaneous analysis of a large number of variables.

This results in a vast amount of data to be processed, analyzed, visualized, and interpreted in the most informative way possible to extract maximum information about the biological process or biological system under study.

From a biostatistical perspective, data obtained through "omics" approaches involve many variables that need to be analyzed jointly. For example, transcriptomic analyses enable the simultaneous study of the expression of thousands of genes.

Therefore, it is desirable to have powerful biostatistical and bioinformatics tools to process, analyze, and interpret the massive amount of data generated by "omics" approaches.

There are numerous techniques for acquiring "omics" data, such as, for example, mass spectrometry, chromatography, sequencing, spectral cytometry, mass cytometry, flow cytometry, and imaging cytometry, among others.

Cytometry refers to a set of techniques for analyzing a biological sample containing a collection of "biological elements", such as cells, vesicles, or particles, in suspension. Cytometry techniques are indispensable analytical methods for the identification and characterization of a population of cells, vesicles, or particles. Moreover, cytomics can address questions about intracellular or extracellular processes and intercellular interactions because cytomics provides a single-cell view of more complex cellular conglomerates, such as tissues in multicellular organisms or communities in the case of unicellular bacteria, yeast, algae, etc.

The reliability and reproducibility of results are paramount; however, a major source of variation in cytometry lies in data analysis. Conventional cytometry data analysis often involves sequential manual selection (or "gating") of regions of interest, typically in two-dimensional scatter plots or contour plots representing one parameter on each of these axes. This is the case, for example, when analyzing flow cytometry data. Analysis is straightforward with three or four-color immunofluorescence data, but becomes significantly more complex when examining an increasing number of cellular markers, leading to increased variability related to the human operator and issues with reproducibility.

Current cutting-edge cytometry techniques can measure tens, hundreds, or even thousands of parameters, thus generating complex multidimensional datasets that are time-consuming to manually analyze. Manual gating is subjective, labor-intensive, unreliable, and costly.

Segmentation can be automated, with segments defined using software without operator supervision. It may involve artificial intelligence, but the criteria for grouping cytometric events are then no longer understandable by the operator. Alternatively, it may rely on analysis algorithms.

Several comparative studies have evaluated unsupervised clustering methods based on their ability to replicate manual gating, detect rare cell populations, and their execution time. While some methods may be effective for certain uses, none meet all the requirements to be used confidently in routine by users.

Furthermore, automated techniques allow cytometric analysis without manual variability, subjectivity, and gating bias, and therefore new methods have been developed in this field over the last decade. However, many automated techniques still require algorithm parameterization requiring operator intervention, introducing a substantial amount of human subjectivity and thus result non-reproducibility.

In the specific example of flow cytometry, the biological fluid containing the cells, vesicles, or particles to be analyzed flows continuously in front of a detector. For each cell, vesicle, or particle passing in front of the detector, it is possible to measure values for cytometric parameters.

In the case of flow cytometry, a "cell event" is a vector associated with a particle and gathers the measured values of cytometric parameters for that particle in an ordered manner. Typically, a cytometric event includes, for each particle, values for more than 10 cytometric parameters. Each value of a cytometric parameter for a particle is called a "cytometric measurement".

To analyze all cytometric measurements, dots representing each cell event can be plotted in two or three dimensions on "presentation graphs". Each dimension corresponds to a cytometric parameter. The graphs representing cytometric events are thus "projections" that allow visualization of two or three cytometric measurements for each cell event (i.e.; cytometric event in this example).

To facilitate analysis, it is necessary to "segment" the dot clouds, meaning to create groups or "segments" gathering cell events with similar values for several cytometric parameters. Analyzing the segments obtained, such as analyzing their respective weights, then allows characterization of the biological fluid, for example, to detect a biological anomaly or pathological risk.

When segmentation is done manually, the user defines the outlines of the segments on the presentation graphs (e.g. scatter plots).

Replacing manual segmentation with unsupervised automatic segmentation remains a major challenge in the field of cytometry. The following four criteria are generally studied to evaluate the quality of an automatic segmentation method: the correlation with manual segmentation, considering all dimensions simultaneously, the segmentation hierarchy and the explicability and robustness of the algorithm. For example, the Applicant has proposed an unsupervised automatic segmentation in Internation patent application WO 2023/094625, which satisfies these requirements.

Even if recent advancement in data generation and automated clustering techniques allow to gather and organize efficiently data, the sheer volume and complexity of this data pose significant challenges in effectively communicating insights to end-users. Despite the strides made in automation, the need for intuitive and interactive data visualization tools remains paramount. There exists a pressing demand for innovative solutions that facilitate user-friendly exploration of this multifactorial data without the need for a priori knowledge of specific cell populations or biological object populations in the sample under analysis. Such tools must empower researchers and biologists to navigate vast datasets effortlessly, uncovering meaningful patterns and relationships hidden within the intricate landscape of biological data. The development of intuitive, interactive visualization platforms represents a crucial frontier in bridging the gap between cutting-edge analytical techniques and practical insights gleaned from cytometry experiments.

## SUMMARY

This invention thus relates to a computer-implemented method visualization (and optionally for clustering) of single cell-type data associated with a plurality of biological objects selected from cells, cellular origin vesicles, microorganisms, acellular microorganisms, particles, and/or biofunctionalized materials; said single cell-type data comprising N events, each associated with a biological object, each cell event being defined by at least two biological object parameters measured for the corresponding biological object.

Said method comprises:

- obtaining at least one hierarchical structure representative of a plurality of classes of biological objects $C_{kh}$ and mutual relationships between said plurality of classes $C_{kh}$ of biological objects; wherein said at least one hierarchical structure, obtained applying a hierarchical clustering method on said single cell-type data, comprises a plurality of classes $C_{kh}$ and associated levels $L_k$, wherein each class $C_{kh}$ is associate to at least one cell event and is representative of at least one biological object and each level $L_k$ is representative of a different degree of similarity among said classes $C_{kh}$;

- rendering a graphical user interface on a visual display device of a system;

- rendering, on the graphical user interface, a graph panel comprising:

    o at least one first graph being a bi- or tri-dimensional scatter plot graphically showing at least a portion of cell events with respect to two biological object parameters, so that said portion of events is represented by a cloud of dots in a 2- or 3-dimensional plot;
    o an interest class selection component for selecting at least one interest class $C_{kh}^I$ of the at least one hierarchical structure;
    o a classes manipulation component for selecting a manipulation to apply to said at least one selected interest class $C_{kh}^I$, said manipulation comprising using the hierarchical structure for:

        ▪ obtaining at least one relationship between said at least one selected interest class $C_{kh}^I$ and at least one among the remaining plurality of classes $C_{kh}$, or
        ▪ obtaining the selected interest class $C_{kh}^I$;

- receiving at least one selected interest class $C_{kh}^I$ and at least one selected manipulation to apply;

- based on said at least one selected interest class $C_{kh}^I$ and said at least one selected manipulation, determining at least one related class $C_{kh}^V$ and associated cell events to be visualized;

- updating the rendering of said graph panel so that the at least one first graph shows at least the associated cell events associated to said at least one related class $C_{kh}^V$.

Advantageously the method of the present invention enables researchers and biologists to efficiently explore extensive datasets, such as the single-cell data, revealing valuable patterns and relationships concealed within complex data. Notably, the components for selection and manipulation of classes, allowing to easily interact with the hierarchical structure, enable the user to intuitively change the granularity of data representation both by magnify and/or reduce the level of data granularity. Thanks to the combined use of these components for selection and manipulation of classes and the hierarchical structure, the used can now explore the data without needing to know in advance for what population to look for.

According to other advantageous aspects of the invention, the computer-implemented method comprises one or more of the features described in the following embodiments, taken alone or in any possible combination.

According to one embodiment, the manipulation consists in determining the one or more upper level classes associated to the selected at least one interest class $C_{kh}^I$ in the hierarchical structure. Advantageously, this manipulation allows to fold the hierarchical structure.

According to one embodiment, the manipulation consists in determining the one or more lower level classes associated to the at least one interest class $C_{kh}^I$ in the hierarchical structure. Advantageously, this manipulation allows to unfold the hierarchical structure in correspondence and at the level of the at least one interest class $C_{kh}^I$.

According to one embodiment, the manipulation consists in receiving at least two selected interest class $C_{kh}^I$ and merging them. Notably, the user may select these selected interest class(es) $C_{kh}^I$ from the at least one first graph using the interest class selection component and then select with the classes manipulation component the action of merging them.

According to one embodiment, the graph panel further comprises a second graph, obtained from the received hierarchical structure, graphically showing said mutual relationships between said plurality of classes $C_{kh}$

of biological objects and said associated levels $L_k$ and wherein updating the rendering of said graph panel further comprises updating the rendering of said second graph so as to graphically shows the determined at least one related class $C_{kh}^V$.

According to one embodiment, said second graph is a sunburst chart comprising concentric rings, each ring representing a level $L_k$ and having a diameter increasing as it represents a lower hierarchical level, and wherein the classes $C_{kh}$ at a level are fractions of the corresponding ring at that level $L_k$.

According to one embodiment, the second graph is a dendrogram representation.

According to one embodiment, the interest class selection component for selecting at least one interest class $C_{kh}^I$ is configured to:

- delineate a region of interest in the at least one first graph comprising a plurality dots, said plurality of dots being associated to at least one class $C_{kh}$;
- calculating a centroid for each sub-ensemble of said plurality of dots associated to one same class $C_{kh}$ comprised in said region of interest;
- using each calculated centroid, identifying in the hierarchical structure the at least one interest class $C_{kh}^I$ to which are associated the dots comprised in said region of interest.

According to one embodiment, said interest class selection component is configured to select the at least one interest class $C_{kh}^I$ from the second graph by means of a user interface.

According to one embodiment, the graph panel further comprises at least one level visualization component configured to select a visualization level $L_k^V$ and, when a visualization level $L_k^V$ is received, the method is further configured to:

- determine, using the hierarchical structure, at least one visualization classes $C_{kh}$ associated to said visualization level $L_k^V$ and the cell events associated to said at least one visualization class;
- update the graph panel rendering so that the second graph shows at least one visualization class $C_{kh}$ and the first graph shows the determined cell events associated to said at least one visualization class $C_{kh}$.

The present invention also relates to a system for visualization (and optionally for clustering) of single cell-type data, the system comprising a visual display device, a user interface, and at least one computer processor coupled to the visual display device and the user interface programmed to perform the method according to any one of embodiments here above.

In addition, the disclosure relates to a computer program comprising software code adapted to perform a method for clustering and visualization compliant with any of the above execution modes when the program is executed by a processor.

The present disclosure further pertains to a non-transitory program storage device, readable by a computer, tangibly embodying a program of instructions executable by the computer to perform a method for clustering and visualization, compliant with the present disclosure.

Such a non-transitory program storage device can be, without limitation, an electronic, magnetic, optical, electromagnetic, infrared, or semiconductor device, or any suitable combination of the foregoing. It is to be appreciated that the following, while providing more specific examples, is merely an illustrative and not exhaustive listing as readily appreciated by one of ordinary skill in the art: a portable computer diskette, a hard disk, a ROM, an EPROM (Erasable Programmable ROM) or a Flash memory, a portable CD-ROM (Compact-Disc ROM).

## DEFINITIONS

In the present invention, the following terms have the following meanings:

The terms **"adapted"** and **"configured"** are used in the present disclosure as broadly encompassing initial configuration, later adaptation or complementation of the present device, or any combination thereof alike, whether effected through material or software means (including firmware).

The term **"processor"** should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

**"Cells"** can come from a unicellular or multicellular organism, of prokaryotic or eukaryotic origin, of animal, plant, fungal, protist, bacterial, or archaeobacterial origin. They can be living, dead, or fixed. Cells may, for example, originate from solid or liquid tissues (such as bone marrow, blood, or lymph, etc.), or from body fluids (such as cerebrospinal fluid, urine, bronchoalveolar fluid, etc.). They can also come from environmental sampling (soils, water etc.).

During the process of acquiring single-cell cytometric data, cells, vesicles, or particles may be in suspension (aqueous or non-aqueous, biological or synthetic), or they may be immobilized on a solid support (for example, a flask, a cell culture dish with one or more wells, a plate or microplate, a glass slide, a chip, etc.). They may have undergone pretreatment with one or more biological or chemical agents.

The cells, vesicles, or particles to be analyzed may be present within one or more populations.

A **"population"** is a set of cells, vesicles, or particles exhibiting identical or similar characteristics. A population can be subdivided into different subpopulations exhibiting different secondary characteristics from each other.

**"Cell-derived vesicles"** are vesicles composed of a membrane of cellular origin. These may include intracellular or extracellular vesicles. Extracellular vesicles are typically secreted or excreted by a cell. **"Cell-derived vesicles"** include, for example, exosomes, ectosomes, microvesicles, microparticles, prostasomes, oncosomes, matrix/calcification vesicles, apoptotic bodies, and other subsets of cellular vesicles.

**"Acellular microorganisms"** refer to microscopic organisms whose structure is not cellular. 'Acellular microorganisms' include, for example, viruses, viroids, and prions.

**"Bio-functionalized materials"** or **"functionalized biomaterials"** or **"bio-functionalized devices"** or **"bio-functionalized systems"** refer to any type of object comprising a material of synthetic or biological origin coated on its surface, coupled, or linked to one or more molecule(s) or functional group(s) of biological origin.

As non-limiting examples, the material of synthetic or biological origin could be a nanoparticle (such as a nanobead, nanosphere, or nanocapsule), a microparticle (such as a microbead, microsphere, or microcapsule), a lipid vesicle such as a unilamellar vesicle, a multilamellar vesicle, a lipoplex, a polyplex, a lipopolyplex, a liposome, a niosome, a cochleate, a virosome, an immunostimulant complex (ISCOM®), etc.

Nanoparticles and microparticles, such as beads, spheres, or capsules, can be organic, inorganic, magnetic, or radioactive. For example, nanoparticles or microparticles can be made of metal, silica, alumina, titanium, glass, ceramic, polystyrene, polymethylmethacrylate, melamine, polylactide, latex, dextran, oxide, graphene, magnetic material, radioactive material, or a combination thereof.

As non-limiting examples, to be bio-functionalized, the material can be conjugated with, or coated with, one or more peptide(s), protein(s), antibody(ies), antibody fragment(s), receptor(s), cytokine(s), chemokine(s), toxin(s), oligonucleotide(s), colored or fluorescent molecule(s), amine, carboxyl, or hydroxyl group(s), bioactive molecule(s) (such as an immunomodulatory molecule, a small chemical molecule, a peptidomimetic, a drug), biotin molecule(s), avidin molecule(s), or streptavidin molecule(s), or a combination thereof.

In the context of the present invention, the term **"cytometric",** when qualifying data, parameters, measurements, events, may refer to cells, cell-derived vesicles or components, or to particles such as acellular microorganisms or bio-functionalized materials.

## BRIEF DESCRIPTION OF THE DRAWINGS

The present disclosure will be better understood, and other specific features and advantages will emerge upon reading the following description of particular and non-restrictive illustrative embodiments, the description making reference to the annexed drawings wherein:

**Figure 1** is a block diagram representing schematically a particular mode of a system, compliant with the present disclosure;

**Figure 2** is a first representation of a graphical user interface comprising a graph panel, the graph panel comprising a first graph, an interest class selection component and classes manipulation component, compliant with the present disclosure;

**Figure 3** is a second representation of the graphical user interface comprising a graph panel, said graph panel comprising a first graph, a first example of second graph, an interest class selection component and classes manipulation component, compliant with the present disclosure;

**Figure 4** is a third representation of the graphical user interface comprising a graph panel, said graph panel comprising a first graph, a second example of second graph, an interest class selection component and classes manipulation component, compliant with the present disclosure;

**Figure 5** is a representation of a portion of the graph panel comprising a first graph and the second example of second graph, compliant with the present disclosure;

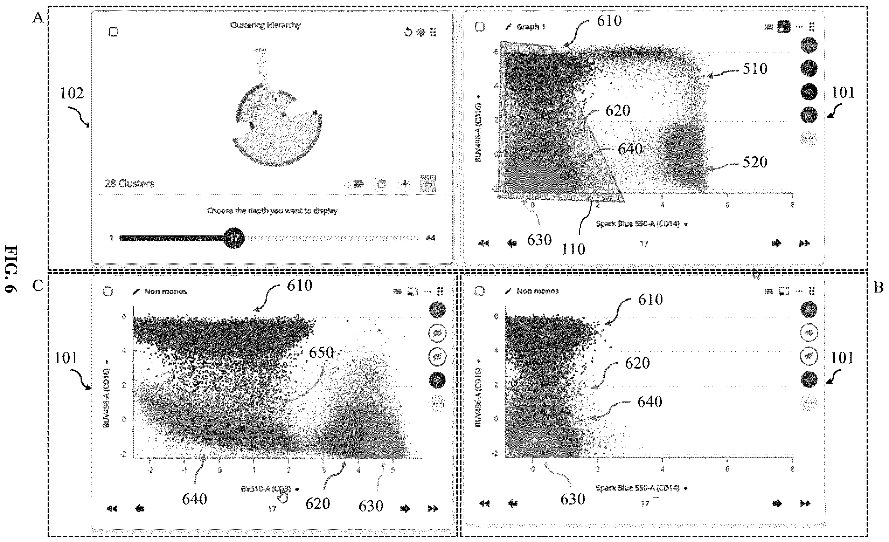

**Figure 6** is a representation of a "lasso" interaction obtained with one embodiment of the present invention compliant with the present disclosure. Panel A show a portion of the graph panel comprising a first graph and the second example of second graph, wherein a region of interest has been defined on the first plot. Panel B shows the updated rendering obtained by applying to the selected clouds of dots the manipulation of represent only these selected classes and panel C shows the updated rendering obtained by applying to the selected clouds of dots the manipulation of represent only these selected classes on a scatter plot with different parameters for the axis;

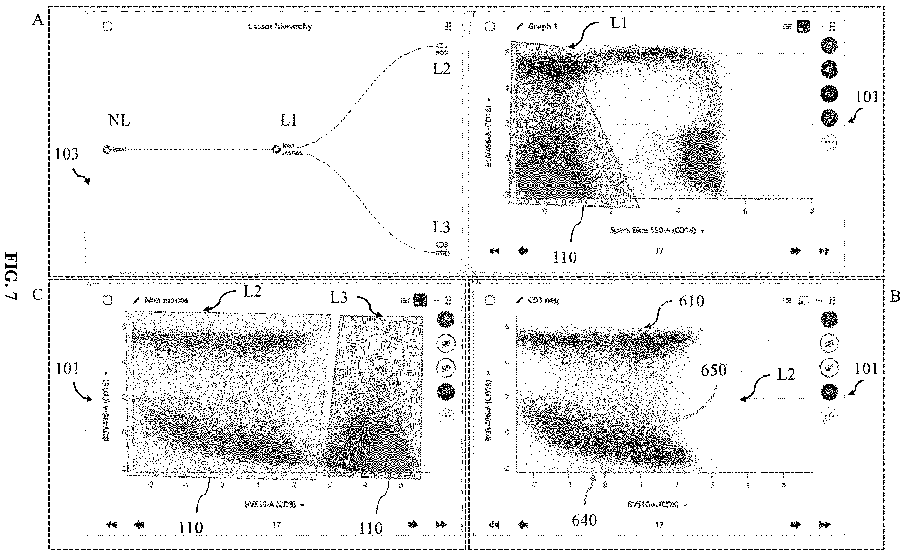

**Figure 7** is a second representation of "lasso" interaction obtained with one embodiment of the present invention compliant with the present disclosure. Panel A show a portion of the graph panel comprising a first graph, where the region of interest has been defined, and a third graph (on the left) to represent the hierarchy between the ROIs defined by the user at the same time. Panel B shows the updated rendering obtained by applying to the selected clouds of dots the manipulation of represent only these selected classes and panel C shows a scatter plot on which two regions of interest have been defined.

**Figure 8** is an illustrative example of the "unfolding" manipulation using the second graph, compliant with the present disclosure;

**Figure 9** is an illustrative example of the "folding" manipulation using the second graph, compliant with the present disclosure;

**Figure 10** is an illustrative example wherein the interest class selection component 110 is configured to select on the first graph one cloud of dots and drop-down menu is used to select the manipulation, compliant with the present disclosure;

**Figure 11** is an illustrative example wherein the graph panel 100 further comprises at least one level visualization component in the form of a slider, compliant with the present disclosure;

**Figure 12** is an illustrative example of the "unfolding" manipulation using the unfold component, compliant with the present disclosure;

**Figure 13** is an illustrative example of the "fast unfolding" manipulation using the fast unfold component, compliant with the present disclosure;

**Figure 14** is an illustrative example of the "fast folding" manipulation using the fast fold component, compliant with the present disclosure;

**Figure 15** is another illustrative example of the "fast unfolding" manipulation using the fast unfold component, compliant with the present disclosure;

**Figure 16** is another illustrative example of the "fast folding" manipulation using the fast fold component, compliant with the present disclosure;

**Figure 17** is an illustrative example wherein the graph panel 100 further comprises a quantitative values information graph, compliant with the present disclosure.

**Figure 18** is a flow chart illustrating the steps of a method for clustering and visualization of single-cell type data associated with a plurality of biological objects, compliant with the present disclosure;

On the figures, the drawings are not to scale, and identical or similar elements are designated by the same references.

## ILLUSTRATIVE EMBODIMENTS

The present description illustrates the principles of the present disclosure. It will thus be appreciated that those skilled in the art will be able to devise various arrangements that, although not explicitly described or shown herein, embody the principles of the disclosure and are included within its scope.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Thus, for example, it will be appreciated by those skilled in the art that the block diagrams presented herein may represent conceptual views of illustrative circuitry embodying the principles of the disclosure. Similarly, it will be appreciated that any flow charts, flow diagrams, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

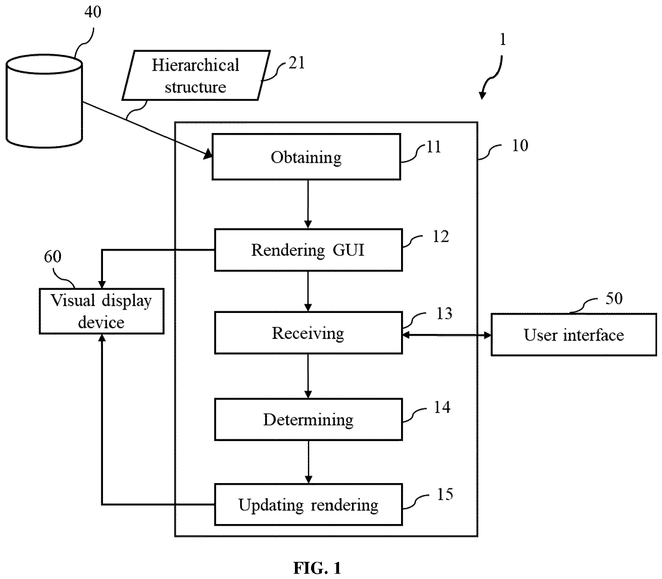

The present disclosure will be described in reference to a particular functional embodiment of a system 1 for clustering and visualization of single cell-type data associated with a plurality of biological objects, as illustrated on **Figure 1.**

The system 1 is adapted to produce a graphical user interface via a visual display device 60 wherein the rendering of a graph panel 100 is updated in real time on the base of the interaction of a user with said graphical user interface by mean of a user interface 50. The system 1 is notably configured to for clustering and visualization of single cell cytometry data associated with biological objects via said graphical user interface.

The system 1 comprises said user interface 50, via which information can be entered by a user. The user interface 50 includes any means appropriate for entering, information or instructions, notably visual, tactile and/or audio capacities that can encompass any or several of the following means as well known by a person skilled in the art: a keyboard, a trackball, a mouse, a controller, a touchpad, a touchscreen, a voice recognition system and the like.

The input data may be derived from at least one hierarchical structure 21 representative of the different biological object populations present in the single cell cytometry data. Alternatively, the single cell cytometry data may be an input data for the system.

More in details, the biological objects, represented in the single cell cytometry data and therefore in the hierarchical structure 21, are selected from cells, cellular origin vesicles, microorganisms, acellular microorganisms, particles, and/or biofunctionalized materials.

In one embodiment, the system 1 for clustering and visualization of single cell cytometry data may be associated with a device for generating the hierarchical structure 21 to be used for cluster manipulation and visualization in system 1, which will be subsequently described.

Though the presently described the system 1, and optionally device for generating the hierarchical structure 21, is/are versatile and provided with several functions that can be carried out alternatively or in any cumulative way, other implementations within the scope of the present disclosure include devices having only parts of the present functionalities.

The system 1 is advantageously an apparatus, or a physical part of an apparatus, designed, configured and/or adapted for performing the mentioned functions and produce the mentioned effects or results. In alternative implementations, any of the system 1 is embodied as a set of apparatus or physical parts of apparatus, whether grouped in a same machine or in different, possibly remote, machines. The system 1 may e.g. have functions distributed over a cloud infrastructure and be available to users as a cloud-based service, or have remote functions accessible through an API.

The system 1 notably comprises visual display device 60, user interface 50 and at least one computer processor 10. In what follows, the modules are to be understood as functional entities rather than material, physically distinct, components. They can consequently be embodied either as grouped together in a same tangible and concrete component, or distributed into several such components. Also, each of those modules is possibly itself shared between at least two physical components. In addition, the modules are implemented in hardware, software, firmware, or any mixed form thereof as well. They are preferably embodied within at least one processor 10 of the system 1.

The processor(s) 10 comprises a module 11 for obtaining (i.e. receiving) at least one hierarchical structure 21 stored in one or more local or remote database(s) 40. The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk). In advantageous embodiments, the at least one hierarchical structure 21 have been previously generated by a system including the device for generating the hierarchical structure 21. Alternatively, the at least one hierarchical structure 21 are received from a communication network.

In one alternative embodiment, the module 11 for obtaining is configured for receiving single cell cytometry data associated with a plurality of biological objects stored in one or more local or remote database(s) 40. In this alternative embodiment, the module 11 for obtaining is further configured to obtain (i.e., calculate) the at least one hierarchical structure 21 representative of a plurality of classes of biological objects $C_{kh}$ and mutual relationships between said plurality of classes $C_{kh}$ of biological objects.

According to the present invention, the biological objects can be:

Cells of animal, plant, fungal, protist, bacterial, or archaeobacterial origin,

Cell-derived vesicles chosen from exosomes, ectosomes, microvesicles, microparticles, prostasomes, oncosomes, matrix/calcification vesicles, or apoptotic bodies,

Acellular microorganisms chosen from viruses, viroids, and prions, and/or

Bio-functionalized materials comprising a material of synthetic or biological origin chosen from a nanoparticle (such as a nanobead, nanosphere, or nanocapsule), a microparticle (such as a microbead, microsphere, or microcapsule), a lipid vesicle (such as a unilamellar vesicle, a multilamellar vesicle, a lipoplex, a polyplex, a lipopolyplex, a liposome, a niosome, a cochleate, a virosome, an immunostimulant complex (ISCOM®), said material of synthetic or biological origin being coupled with, or coated with, one or more peptide(s), protein(s), antibody(ies), antibody fragment(s), receptor(s), cytokine(s), chemokine(s), toxin(s), oligonucleotide(s), colored or fluorescent molecule(s), amine, carboxyl, or hydroxyl group(s), bioactive molecule(s) (such as an immunomodulatory molecule, a small chemical molecule, a peptidomimetic, a drug), biotin molecule(s), avidin molecule(s), or streptavidin molecule(s), or a combination thereof.

Single cell cytometry data can be obtained by any method capable of analyzing, determining, or measuring characteristics of a cell, vesicle, or particle. The analysis may involve a sample comprising a plurality of cells, vesicles, and/or particles. For example, Single cell cytometry data (also called cytometry data in the present description) can be obtained from a biological sample from an individual, such as one or more organ(s), tissue(s), cell(s), or cell fragment(s) from the individual.

In some cases, the method of acquiring cytometric data may be preceded by a sample preparation process, which may include, for example, a step of isolating the cells, vesicles, and/or particles, or prior isolation of the components of said cells.

The sample preparation process involving the biological objects associated with the cytometric data may include, as non-limiting examples, one or more step(s) of magnetic-activated cell sorting (or "MACS"), laser capture microdissection (or "LCM"), manual cell harvesting or micromanipulation, microfluidics, limiting dilution, optical trapping separation, electrophoresis, bead separation, immunoprecipitation, immunopanning, labeling, immunostaining, immunofluorescence, multiplexing, and/or the use of biochips.

Cytometric data can be data from "omics" or "meta-omics" technologies, for example, genomic, epigenomic, transcriptomic, proteomic, metabolomic, lipidomic, etc., data. Thus, cytometric data can, for example, relate to the detection and/or quantification of DNA (e.g., genes), RNA, proteins (e.g., cytokines, receptors...), sugars, amino acids, and/or fatty acids present in or on the surface of the cell, vesicle, or particle, or of any other molecule contained within the cell, vesicle, or particle. Cytometric data 21 can also relate to the detection of the presence or absence of a particular molecule or assembly in or on the surface of the cell, vesicle, or particle, or to the detection of interactions between molecules present in or on the surface of the cell, vesicle, or particle, or to the detection of interactions between cells and/or vesicle-like objects, microorganisms, or bio-functionalized materials.

As non-limiting and purely illustrative examples, when cytometric data are of proteomic type, the measured or analyzed cytometric parameters may be the expression level of one or more intracellular or extracellular proteins, or the expression level of one or more receptors or markers on the surface of the cell, vesicle, or particle.

Other examples of cytometric parameters include the size of cells, vesicles, or particles, their density, granularity, morphology/shape, refractive index, membrane composition, molecular content (such as the presence or absence of an intracellular or surface molecule) or their content (such as intracellular content or content of ions, DNA, RNA, ...), or the redox state of the cell, its status in the cell cycle, apoptotic state, or phosphorylation level...

Cytometric data can be obtained through single-cell analysis technologies.

As non-limiting examples, cytometric data can be obtained by flow cytometry (or FACS for "fluorescence activated cell sorting"), by PCR-activated cell sorting (or PACS), by microsphere affinity proteomics (MAP), by mass spectrometry, by chromatography, by CYTOF, by spectral cytometry, by mass cytometry, by imaging cytometry, by gene expression microarrays, by sequencing, for example of DNA ("DNA-seq" or "single cell (sc)DNA-seq") or RNA ("RNA-seq" or "single cell (sc)RNA-seq"), by in situ hybridization, and/or by microscopy. The term "microscopy" notably refers to atomic force microscopy (AFM), electrochemical detection (EC), scanning electron microscopy (SEM), transmission electron microscopy (TEM), surface plasmon resonance imaging (SPRi), Raman microspectroscopy...

Cytometric data can also be obtained by combining several of these techniques. As a purely illustrative example, multiplex profiling of RNA and protein expression at the single-cell level can be obtained simultaneously by combining the PLAYR technique (proximity ligation assay for RNA), which allows measurement of the expression level of a large number of RNAs by flow or mass cytometry, with a technique for detecting surface or internal proteins labeled with antibodies.

Another purely illustrative example of a technique for obtaining cytometric data is the profiling of individual cells isolated in liquid droplets, enveloped by a thin semipermeable membrane (microcapsules), by high-throughput RNA cytometry, for example by multiplexed RT-PCR.

According to the alternative embodiment wherein the at least one hierarchical structure is generated from the processor(s) 10, said hierarchical structure(s) may be obtained using a hierarchical clustering method on said single cell-type data. In one embodiment, the single cell-type data are single-cell data. In one example, said hierarchical structure(s) may be obtained using the method disclosed in patent application WO 2023/0946625. Other methods may be used for the obtention of hierarchical structure(s) such as agglomerative clustering, flowsom, k-means and the like.

Notably said hierarchical structure(s) is characterized in that it comprises a plurality of classes $C_{kh}$ and associated levels $L_{k}$, wherein each class $C_{kh}$ is associated to at least one event and is representative of at least one biological object and each level $L_{k}$ is representative of a different degree of similarity among said classes $C_{kh}$. Advantageously, the use of a hierarchical structure 21 to visualize and explore the data allows the user to fumble the single cell-data without needing to "know what to search for" from the beginning of the analysis. Indeed, in the classical systems for cytometry data analysis/visualization the user needs to have already an idea of the populations he/she is interested to, as he/she has to select iteratively the populations by defining windows (i.e., lasso) on the scatter plot(s) and may only easily zoom-in the data, but difficultly take a step back.

In the present invention, an "event" is a vector associated with a biological object and gathers the measured values of biological object parameters in an ordered manner. Typically, an event includes, for each biological object, values for more than 10 biological object parameters.

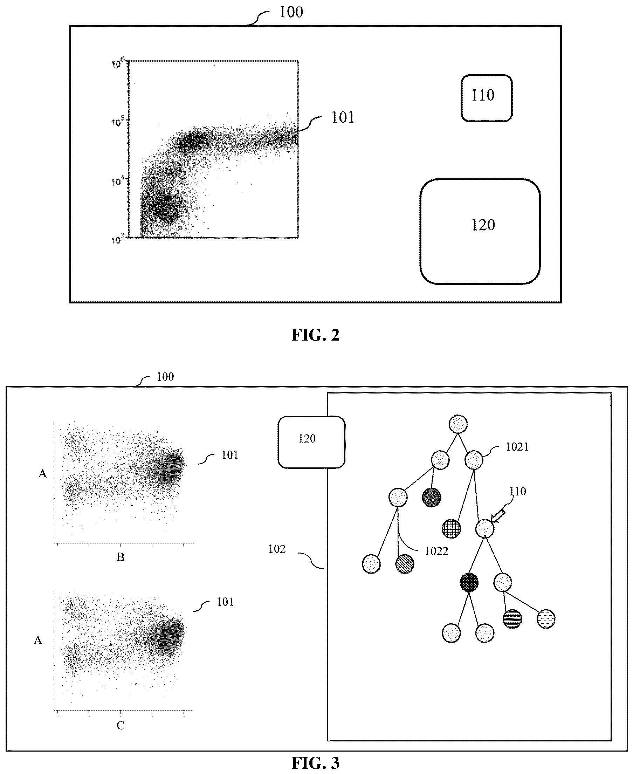

The processor 1 further comprises a module 12 for rendering the graphical user interface on the visual display device 60 of the system 1. Notably, the module 12 is further configured to perform rendering, on the graphical user interface, a graph panel 100, as illustrated in **Figure 2.** Said graph panel 100 comprises at least one first graph 101, which may be a bi- or tri-dimensional scatter plot configured to graphically show at least a portion of events with respect to one to three biological object parameters. Indeed, as will be detail below in the disclosure and with reference to the case of single-cell data, due to the large number of events comprised in one set of single cell cytometry data, the user does not visualize all data at once but only portions of it. This kind of representation of single cell-type data take the form of a cloud of dots in a 2- or 3-dimensional plot. Indeed, scatter plots are commonly used to visualize the relationship between two or more biological object parameters. Each data point (i.e., dot) in the scatter plot represents a single cell or biological object (i.e., event), and its position on the plot corresponds to the values of the parameters being compared. This is therefore a representation to which biologists specialized in single cell data analysis are particularly used to. In one example, the graph panel comprises two or more first graphs 101. One or more of the first graph(s) 101 may be a N-dimensional scatter plot, for N superior to two. Indeed, for cytological data with three or more dimensions, 3D scatter plots can be used to visualize relationships between multiple parameters. These plots provide a more comprehensive view of the data but can be challenging to interpret in complex datasets.

In the scatter plot, each data point (i.e., event) may be represented with an appearance (i.e., visual representation) depending on which class(es) $C_{kh}$ they belong to and, optionally, the selection/manipulation that the user has performed within the graph panel 100. The appearance of the dots may be determined by their shape (i.e., shape of the icon, such as a full cercle, hole cercle, triangle, point, star, and the like), dimension, color and any other visualization parameter that may be suitable to discriminate clusters of dots on a scatter plot.

The graph panel 100 may comprise any type of interacting component allowing the user to choose the number of scatter plots that he/she wants to display at the same time, the dimensions of one scatter plot (e.g., 2D or 3D), the biological object parameters to be represented on the axis, the appearance of the dots associated with one class and any other functionality known in the domain of the visualization of single cell-type data. In one embodiment, the graph panel 100 may comprise the graph 1D such as a histogram.

The graph panel 100 may comprise other type of graphs (i.e., different visualization techniques) than first graph 101, as representing simultaneously different visualization of the cytological data advantageously offers a unique insight into the structure and characteristics of the data and can help researchers interpret their findings more effectively. Advantageously, representing simultaneous at least two graphs of different type, such as one scatter plot and one sunburst chart, allows the user to easily and intuitively navigate the single cell-type data while always knowing at which granularity of representation is currently visualized these data (i.e., the depth in the level(s) of the hierarchical structure that is currently visualized). These other type of graphs (102, 103, 104), and the possible ways to interact with them, will be described in detail below.

In one embodiment illustrated in **Figure 2,** the graph panel 100 further comprised two components 110 and 120 configured to allow a user (i.e., biologist, researcher etc.) to navigate/manipulate the cytological data that have been previously organized in said hierarchical structure 21. This navigate/manipulate the cytological data allows the user to perform his analysis of single cell-type data (e.g., cytological data).

The component 110 is called in the present disclosure "interest class selection component" as it is configured for selecting at least one interest class $C_{kh}^{I}$ of the hierarchical structure(s) 21. The component 110 may be used by the user to select one or more interest classes $C_{kh}^{I}$ of the hierarchical structure(s) 21, notably via a physical interaction with the user interface 50. Examples of how the interest class selection component 110 may works will be provided below.

The component 120 is called in the present disclosure "classes manipulation component" as it is configured for selecting a manipulation (i.e., operation) to apply to the selected interest class $C_{kh}^{I}$ by using the hierarchical structure 21. The manipulations selected by component 120 may be configured to look up into the hierarchical structure 21 to access the relationships between the selected interest class(es) $C_{kh}^{I}$ and at least one of the remaining (non-selected) plurality of classes $C_{kh}$. Examples of how the interest class selection component 120 may works for this type of manipulation will be provided below (e.g., the folding and unfolding of the hierarchical structure). Alternatively, a manipulation selected by component 120 may be configured to obtaining the selected interest class $C_{kh}^{I}$, like in the example of the "lasso" that will be described in detail below. Advantageously the components (e.g. 110, 120) allow the user to easily explore, and therefore exploit, this huge amount of multifactorial data. As will be illustrated by the examples, the selection of class(es) and manipulations (and the consequent update of the rendering) allows the user to interactively grope in the data, without need of a previous knowledge of the existing populations in the analyzed sample, by changing the granularity of the data visualization in both directions (i.e., zoom in the data so as to see more and more sub-populations or zoom out to only visualize few not-specific populations). This intuitive exploration is made possible notably thanks to the hierarchical structure 21 and these selection and manipulation components (e.g., 110,120) allowing to exploit it.

According to the present invention, the graph panel 100 may comprise at the same time as much interest class selection components 110 and as much classes manipulation components 120 as the embodiments described in the present description, as they all are components (110, 120) that may coexist simultaneously on the graph panel 100.

The device 1 further comprises a module 13 for receiving at least one selected interest class $C_{kh}^{I}$ and at least one selected manipulation to apply. The at least one selected interest class $C_{kh}^{I}$ may be notably received as consequence of an interaction between the user and the interest class selection component 110, via the user interface 50. The at least one selected manipulation to apply may be notably received as consequence of an interaction between the user and the classes manipulation component 120, via the user interface 50.

The device 1 further comprises a module 14 for determining which is configured to determine the one or more (related) class $C_{kh}$, and therefore the events associated to the one or more (related) class $C_{kh}$, which are related (i.e., directly or indirectly connected in the hierarchical structure 21) to the selected interest class(es) $C_{kh}^{I}$ according to the selected manipulation.

The device 1 further comprises a module 15 for rendering updating which is configured to update the rendering of the graph panel 100 so that the at least one first graph 101 shows the determined events associated to said at least one related class $C_{kh}$.

In following description will provide different embodiments that the system 1, and notably processor 10, may be configured to implement, alone or in combination.

In one example illustrated in **Figure** 3, the graph panel 100 comprises more than two first graph 101, configured to represent events (for example, selected from the user) with respect to different biological object parameters (e.g., A, B, C, D, E in Fig. 3).

The graph panel 100 may as well comprise at least one second graph 102 which is configured to provide a graphical representation of the hierarchical structure 21. The second graph(es) 102 may be obtained from the received hierarchical structure. Indeed, the processor 10 may be further configured to use the hierarchical structure 21 to obtain the second graph(es) 102 and render it in the graph panel 100. The second graph(es) 102 is notably constructed to graphically show the mutual relationships between the plurality of classes $C_{kh}$ of biological objects and said associated levels $L_k$ of the the hierarchical structure 21.

When the graph panel 100 comprises at least one second graph 102, the module 15 for rendering updating is further configured to update the rendering of the at least one second graph 102 so as to graphically shows the determined at least one related class $C_{kh}$.

The second graph 102 may be a dendrogram representation, as illustrated in Figure 3. The dendrogram is a diagram representing a tree commonly used to illustrate the arrangement of groups generated by hierarchical clustering. In the dendrogram, each node 1021 represents a class $C_{kh}$, and the edge 1022 represent hierarchical links between the classes $C_{kh}$. The nodes 1021 belonging to the same level $L_k$ may be aligned in strata.

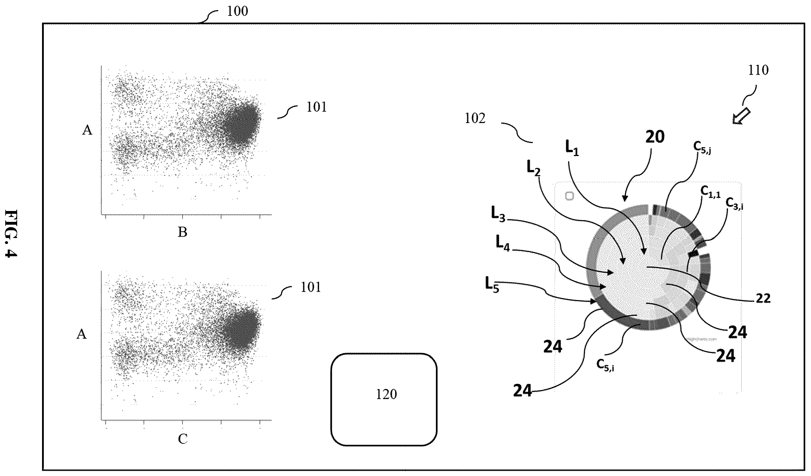

Alternatively, or in complement, the second graph 102 may be a sunburst chart, as illustrated in Figure 4, which is a graph comprising concentric rings, each ring representing a level $L_k$ and having a diameter increasing as it represents a lower hierarchical level, and wherein the classes $C_{kh}$ at a level are fractions of the corresponding ring at that level $L_k$. More in detail, this chart is depicted as a disk divided into a plurality of sectors or "quarters", with each sector representing all, and only the events (and/or associated single cell-type data) of a respective class at the highest level. Preferably, the angle of a sector is proportional to the number of events in the class of the foundation (base) level it represents. The sunburst chart includes concentric rings, each ring representing a level of the hierarchical structure 21. Notably, there is a core associated with level $L_1$ and $l$ - 1 concentric rings each representing other levels $L_k$ for k ranging from 2 to and $l$ - 1 with a hierarchy lower than level

$L_1$. In this sunburst chart, a ring has a diameter that is larger the lower the hierarchical level it represents.

The classes $C_{kh}$ of the same level $L_k$ can be represented as fractions of the corresponding ring, and each fraction extends into the sector containing the classes of higher hierarchical level before a direct or indirect hierarchical link. Two classes have a direct hierarchical link if only one edge connects them in the hierarchical structure 21, and an indirect hierarchical link if there are at least two edges connecting them in the hierarchical structure 21.

A fraction representing a class may notably extend around the center of the disk along an arc whose angle of opening is proportional to the number of events in the class represented by the fraction.

Figure 4 illustrates an example second graph 102 being a sunburst chart, in which the levels are represented by concentric rings 24, with rings closer to the center representing aggregated classes. The classes $C_{kh}$ of a level $L_k$ are fractions of the ring 24 corresponding to that level $L_k$.

In this example, the diameter of a ring 24 is larger the further it represents a level $L_{2-5}$ away from the base level $L_1$.

Preferably, for all levels, the fractions of a ring from a lower hierarchical level (i.e., daughter) representing daughter classes $C_{(k+1)h}$ of a mother class $C_{kh}$ are depicted in the same angular sector as the fraction represents said mother class $C_{kh}$ in the ring associated with the higher hierarchical level (i.e., mother). This way, the fractions of daughter classes $C_{(k+1)h}$ externally overlay the fraction associated with the mother class $Ckh$.

For example, when the analyzed objects are cells, a mother class could correspond to a cellular population of lymphocytes. This lymphocyte population (mother class) can be subdivided into different cellular subpopulations (corresponding to daughter classes of the mother class), such as, for instance, a subpopulation of T lymphocytes and a subpopulation of B lymphocytes, both of these subpopulations having differing secondary cytometric parameters. On the sunburst chart 102, the various cellular subpopulations are represented by a "daughters" ring surrounding the "mother" ring.

Additionally, these T and B lymphocyte subpopulations can themselves be subdivided further. For instance, within the T lymphocyte subpopulation, analysis of cytometric parameters might distinguish a subpopulation of CD4+ T lymphocytes (T lymphocytes expressing the CD4 protein on their surface) and a subpopulation of CD8+ T lymphocytes (T lymphocytes expressing the CD8 protein on their surface). The sunburst chart 102 can depict the entire hierarchical structure 21 or, preferably, only a portion of the hierarchical structure 21.

According to one embodiment, the interest class selection component 110 is configured to select the at least one interest class $C_{kh}^{I}$ from/via the second graph 102 by means of a user interface 50. The interest class selection component 110 may be an on-screen graphical indicator (i.e., a cursor), in the shape of an arrow, hand, or other symbol, representing the position of a user's pointing device, such as a mouse or touchpad (i.e., user interface 50), on the visual display device 60. The interest class selection component 110 therefore allowing the user to interact with the the second graph 102 by selecting, clicking, dragging, or performing other actions on this graphical representation of the hierarchical structure 21.

With reference to the example of the dendrogram of **Figure 3,** the user may move the interest class selection component 110 (e.g., cursor in the form of an arrow) in proximity, or so as to overlap, to one interesting node of the dendrogram and then click so as to select the interest node, and therefore the interest class $C_{kh}^{I}$ of the hierarchical structure 21 represented by the interesting node, that the used wishes to manipulate. The operation of selecting a node of interest may be repeated multiple times so as to select more than one interest class $C_{kh}^{I}$ at once that the user may want to manipulate/visualize at the same times.

With reference to the example of the sunburst chart of **Figure 4,** the user may move the interest class selection component 110 (e.g., cursor in the form of an arrow) in proximity, or so as to overlap, to one interesting fraction of the sunburst chart and then click so as to select the interest fraction, and therefore selecting the interest class $C_{kh}^{I}$ of the hierarchical structure 21 represented by said fraction. As in the precedent case, the operation of selecting a fraction of interest may be repeated multiple times so as to select more than one interest class $C_{kh}^{I}$ at once that the user may want to manipulate/visualize at the same times.

When a hierarchical structure 21 is obtained for the first time from the system 1, a set of initializations rendering parameters may be used for rendering for the graph panel 100 for the first time (i.e., before reception of any selection and manipulation choice from the user). This set of initializations rendering parameters may be predefined and stored in the database 10 to be accessed when needed. For example, the set of initializations rendering parameters may be configured so that all data points are visualized at the first rendering and therefore all fractions available in the sunburst chart are deployed. Alternatively, this set of initializations rendering parameters may be predefined, by the user during a precedent utilization of the system 1, on another hierarchical structure 21, and be stored in the database 10. In another example, this set of initializations rendering parameters may be calculated on the base of the hierarchical structure 21 so as to provide an optimized choice of classes to visualize.

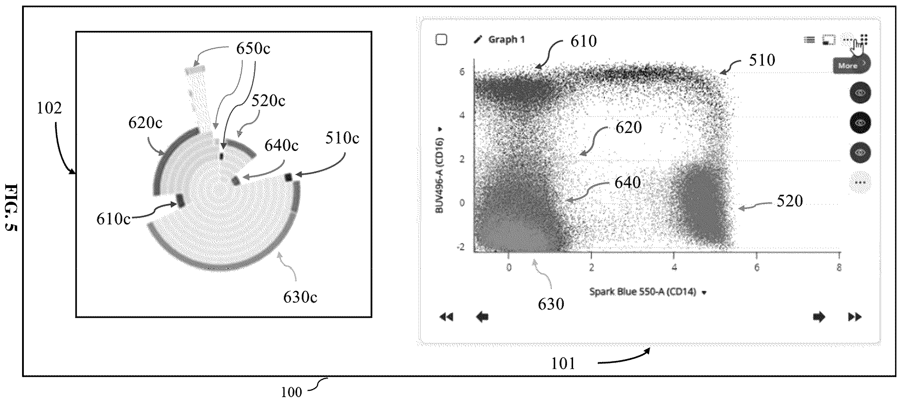

**Figure 5** first provides an example of how graph panel 100 may comprise one second graph 102 representing the hierarchical structure 21 in the form of a sunburst chart and one first graph 101 (i.e., scatter plot). This may be, for example, the first rendering (i.e., obtained using the set of initializations rendering parameters) of a new hierarchical structure 21, just obtained by the system 1, that the user would like to start to analyze. On the sunburst chart 102, the outer fractions, represented in the brightest colors, are the fractions that are currently selected for visualization. The internal fractions and rings, visualized in dimmer colors, represents the architecture of the higher level of the hierarchical structure 21 parent of these fractions currently selected for visualization.

In other words, the sunburst chart 102 represents the classes $C_{kh}^{V}$ currently chosen by the user for visualization plus, in dimmer colors, the underlying portion of the hierarchical structure 21 (i.e., the lower levels of the hierarchical structure not selected for visualization are not represented in the sunburst chart). In the first graph 101, on the left, the corresponding events of the current classes $C_{kh}^{V}$ are represented as dots, rendered with the same color of the corresponding fraction on the sunburst chart 102. This provides to the user at once both a high-level visualization of the hierarchy of the classes visualized and a more granular visualization with the distributions of the events associated to these classes with respect to two biological object parameters (said biological object cell parameters may have been as well chosen by the user).

More in details, on the scatter plot 101 of figure 5 is mainly possible to distinguish 5 clusters of dots (i.e., groups of dots, each group being associated to one class current interest classes $C_{kh}^{I}$ ): cluster 510 representing intermediate monocytes, cluster 520 classical monocytes, and the overlapping clusters 610, 620, 630 and 640 which represents events that are not monocytes. Each of these clusters is a representation on the scatter plot 101 of the events associated to the respective classes represented from the selected fractions (510c, 520c, 610c, 620c, 630c and 640c) on the sunburst chart 102. It is hardly possible to guess that other clusters, associated to the classes $C_{kh}^{V}$ visible on sunburst chart 102 (fractions identified as 650c), are hidden behind the overlapping clusters 610, 620, 630 and 640. In the case scenario of Figure 5, the user may want to modify the rendering of the graph panel 100, notably of the first graph 101, to have a much clearer vision of these not monocytes clusters.

In one embodiment, the interest class selection component 110 is configured to select the interest class

$C_{kh}^I$ by interacting with one first graph 101. Different ways are available to the user to interact with the interest class selection component 110 in order to select the interest class $C_{kh}^I$. For example, the user may move the interest class selection component 110 on one of the first graph 101 rendered and stop it in proximity of one or more dots of a same color, and by clicking in this position, the class associated to the one or more dots is selected as the interest class $C_{kh}^I$.

In one other example, the interest class selection component 110 is configured to determine/design a region of interest (ROI) on the first graph 101 and on the base of the analysis of the dots comprised in said ROI, determine (and select) the interest class $C_{kh}^I$. More in detail, the interest class selection component 110 is configured to firstly delineate a region of interest in the at least one first graph (101) (i.e., "lasso" delineated by means of the user interface 50). This ROI may comprise dots belonging to one or more $C_{kh}^V$ currently visualized on the scatter plot 101 (i.e., one or more clouds of dots visualized in different colours). Once the selection of the ROI from the user is received, the processor(s) 10 calculates a centroid for each of the cloud of dots, comprised in said ROI, that are associated to the one or more $C_{kh}^V$ currently visualized on the scatter plot 101. Then, the calculated one or more centroids are used to identifying the one or more interest classes $C_{kh}^I$ in the hierarchical structure 21 to which are associated the events comprised in said region of interest.

In this particular embodiment of the interest class selection component 110, the component 120 may be configured to obtain the selected interest class $C_{kh}^I$. In this case, the component 120 may allow the user to choose one of the following actions: to visualize the events associated to the selected one or more interest classes $C_{kh}^I$ in a new scatter plot, to remove them from the current scatter plot. The component 120 may be configured to also to obtaining at least one relationship between said at least one selected interest class $C_{kh}^I$ and at least one among the remaining plurality of classes $C_{kh}$. In this case, the component 120 may allow the user to choose one of the following actions: determining the one or more upper level classes in the hierarchical structure 21 associated to the selected interest class(es) $C_{kh}^I$ (i.e., unfolding of hierarchical structure for these selected interest classes but not for the others classes $C_{kh}^V$ displayed on the scatter plot that are not comprised in the ROI), determining the one or more lower level classes in the hierarchical structure 21 which are associated to the interest class(es) $C_{kh}^I$ (i.e., merge them together to their common ancestor but not the others classes $C_{kh}^V$ displayed on the scatter plot that are not comprised in the ROI), changing the display of the events associated to these classes (i.e., change the colour, shape and/or dimension of the dots), launch a new clustering on the events of only these interest class(es) $C_{kh}^I$, extracting these interest class(es) $C_{kh}^I$ and storing them separately for further analysis.

This example of implementation may be described more practically in view of **Figure 6,** where the user places a ROI on the left part of the scatter plot so to select the not monocytes events and leave aside the monocytes events 510 and 520. In a first moment the user only visualizes the second graph 102 and the first graph 101 in panel A. Indeed, the red line 110 represents the region of interest drawn by the user (by means of the user interface 50), which partially encompassing the overlapping clusters 610, 620, 630, 640 and 650 of not monocytes. The determining module 14 is therefore configured to calculate for each cluster of dots (i.e., cloud of dots) comprised in the ROI (610, 620, 630, 640 and underlying 650) its centroid. These calculated centroids are then used to identifying the interest classes $C_{kh}^I$ in the hierarchical structure 21 to which each of the cluster comprised in the ROI is associated. As the interest classes $C_{kh}^I$ are selected is now possible to obtain the ensemble of events associated to each of the interest classes $C_{kh}^I$ by looking up into the hierarchical structure 21. The user may now use the classes manipulation component 120 to choose to represent the events associated to the interest classes $C_{kh}^I$ in a new scatter plot 101. This action may be performed for example using a double click on the region of interest or by choosing the action from a drop-down menu that may be visualized with a right click. This corresponds to apply as manipulation an identity, in other words apply an operation configured to determine that the related classes $C_{kh}^V$ are equal to the interest classes $C_{kh}^I$. Therefore, the determining module 14 provides as output the related classes $C_{kh}^V$ (equal to the interest classes $C_{kh}^I$) and the associated ensembles events to be visualized, one for each related class $C_{kh}^{V}$ .

The updating rendering module 15 may now update the rendering of the graph panel 100, by adding to the information in the panel A, for example the first graph 101 in the panel B and/or the first graph 101 in the panel C, if the user has also chosen to change the biological object parameters for the scatter plot. In the first graph in panel B, the clusters 510 and 520 are removed while the clusters in the ROI are still represented in the scatter plot in their integrality. Thanks to the steps of calculating the centroids and searching from the corresponding classes in the hierarchical structure 21 (i.e., identification step) it is possible to recover the totally of the events belonging to each one of the interest classes $C_{kh}^{I}$ , which advantageously allows the user to visualize in the new scatter plot all events even those he/she could have missed by designing the ROI. As shown in panel A, a portion of the violet dots of the cluster 610 is comprised in the red line of the ROI, however also these left-aside dots are present in the scatter plot of panel B.

As shown in panel C, the user may modify the biological object parameters for representation of the scatter plot in order to find a better representation space where the non-monocytes clusters (610, 620, 630, 640 and underlying 650) are distant and therefore easier to discriminate by the user during analysis. In this example, the sunburst chart 102 does not change at the update of the rendering.

This embodiment of the interest class selection component 110, allowing selection of classes via definition of a ROI (also called "lasso"), may be repeated multiple times so that at least two ROIs may be visible at the same time, on one or more scatter plots, on the graph panel 100 as shown in Figure 7. A second ROI L2 may be as well defined in addition to the first ROI L1, as illustrated in **Figure 7.** This second ROI may be drawn on any of the existing first graphs 101 rendered on the graph panel 100. In this illustrative example, the user has used the interest class selection component 110 to draw the second ROI L2, then a third ROI L3 on the scatter plot in panel C. As described, the determination module 14 calculates the corresponding centroids and ensemble of events to visualize. In this example the user had decided to visualize only the cloud of points selected by the ROI L2 in the scatter plot represented in panel B.

The user may also choose to add to the graph panel 100 a graph 103 (on the left of panel A of Fig. 7) to represent the hierarchy between the ROIs defined by the user at the same time. In the example of Figure 7, it is indeed illustrated that the ROIs L2 and L3 are both daughters of initial ROI L1, which have been selected from the scatter plot of panel A (i.e., NL is the label describing the ensemble of all dots represented in this scatter plot). The user also may for example label the clusters comprised in the region of interest, in other words the graph panel 100 comprises a component allowing the user to enter a string of characters and associated it as a label to the selected clusters of dots.

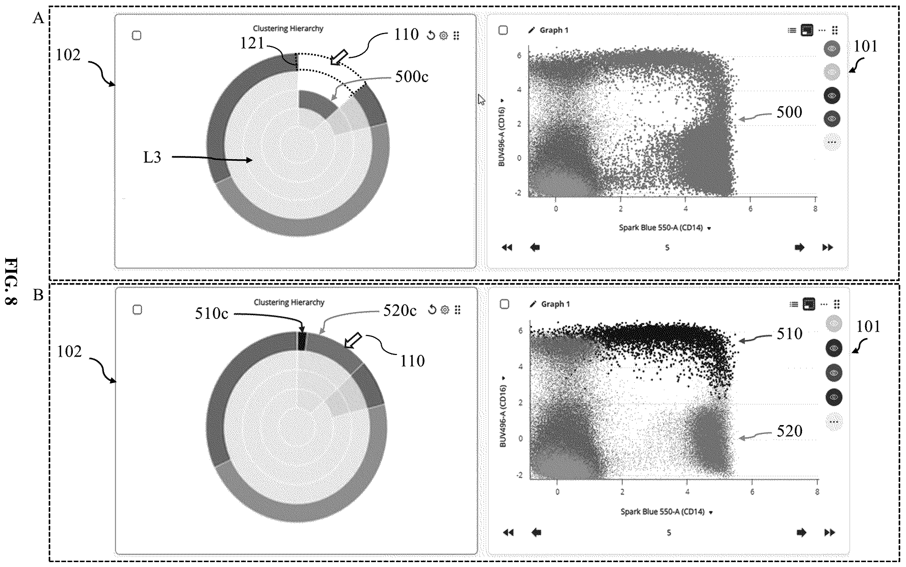

According to one embodiment, the manipulation selected with the component 120 consists in the manipulation consists in finding the one or more lower level classes associated to the at least one interest class $C_{kh}^{I}$ in the hierarchical structure 21. An example of this "unfolding" manipulation is provided in **Figure 8,** wherein panel A represent one sunburst chart 102 and its related scatter plot 101 before applying the "unfolding" manipulation and the panel B represent the updated rendering of sunburst chart 102 and its related scatter plot 101 after the "unfolding" manipulation.

In this example, the user is visualizing on the scatter plot a first cloud of brown dots 500 representing generic monocytes and on the left multiple clouds of dots that the user knows (thanks to his/her general knowledge) to be probably corresponding to non-monocytes cells. The user may now want to see how many types of monocytes may be present in the first cloud of brown dots 500. To do so, he may use the interest class selection component 110 to explore the hierarchical structure 21 by clicking on the "empty" portion of the on the sunburst chart 102 of panel A contoured by a dotted line 121.

In this example, the sunburst chart itself is configured to be the classes manipulation component 120 for selecting a manipulation to apply to said at least one selected interest class $C_{kh}^{I}$ . Indeed, by clicking on the delimitated portion 121, the user has selected: (1) as the interest class $C_{kh}^{I}$ the class associated to the fraction 500c, which is comprised in the same section of the sunburst chart of the delimitated portion 121 and (2) to manipulate the interest class $C_{kh}^{I}$ (500c) by "unfolding" the hierarchical structure from the level associated to the fraction 500c ($L_3$) to the level of the delimitated portion 121 (i.e., $L_5$, same level that the red cluster represented in the scatter plot on the right).

In this example, the determination module 14 is configured to explore the hierarchical structure 21 to determine which are the daughters' classes of the fraction 500c at the level $L_5$ of the hierarchical structure (i.e., determining the at least one related class $C_{kh}^{V}$ ). In this case, the determination module 14 find that the hierarchical structure comprises two related classes $C_{kh}^{V}$ belonging to the same section of the fraction 500c (i.e., daughters' classes) and associated to the level $L_5$. The updating rendering module 15 therefore updates the rendering of the graph panel 100 from panel A to panel B of Figure 8, wherein the sunburst chart is updated to show the fractions 510c and 520c representing the two related classes $C_{kh}^V$ and the scatter plot is updated to show the corresponding two clouds of dots 510 (blue) and 520 (brown). The cloud of dots 510 is associated to the intermediate monocytes and the cloud of dots 520 is associated to classical monocytes. Advantageously, in this way, the underlying structure of the single cell-type data corresponding to the monocytes is revealed and easily visualized to the user.

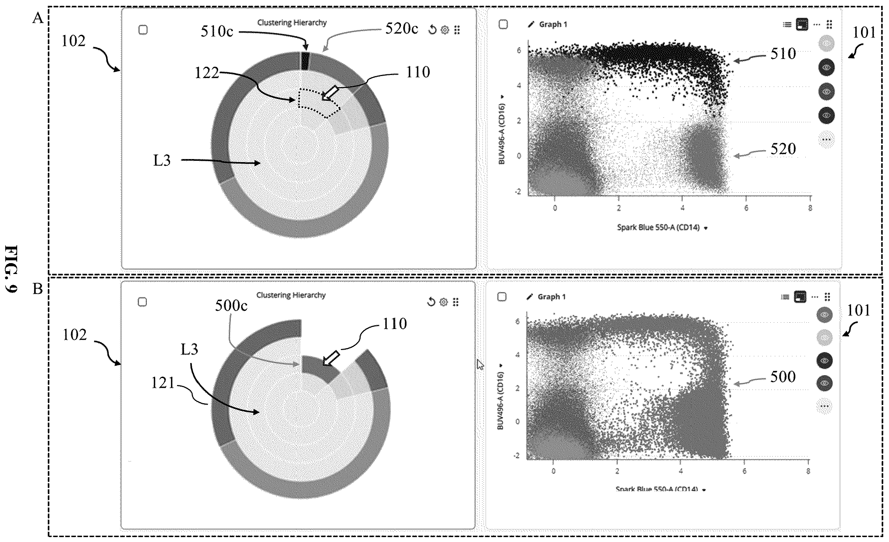

According to one embodiment, the manipulation selected with the component 120 consists in determining the one or more upper level classes associated to the selected at least one interest class $C_{kh}^I$ in the hierarchical structure 21. An example of this "folding" manipulation is provided in **Figure 9,** wherein panel A represent one sunburst chart 102 and its related scatter plot 101 before applying the "folding" manipulation and the panel B represent the updated rendering of sunburst chart 102 and its related scatter plot 101 after the "folding" manipulation.

As in the previous example, the sunburst chart itself is configured to be the classes manipulation component 120 for selecting a manipulation to apply to said at least one selected interest class *Ckh.* Indeed, by clicking using the interest class selection component 110 on the fraction of the sunburst chart 102 enclosed by a dotted line in figure 9 (ref. 122 in Fig. 9, panel A) this fraction, and therefore the corresponding class $C_{kh}^V$, is selected to be the selected interest class *Ckh.* As the selected fraction is in a higher-level ring of the sunburst chart 102, the consequence of this selection on the sunburst (acting as the classes manipulation component 120) is to search in the hierarchical structure 21 the at least one class that is upstream (i.e., a class connected to the selected interest class $C_{kh}^I$ but being on a high level). More precisely, the described action corresponds to search for the one or more classes connected to the selected interest class $C_{kh}^I$ that is at the level $L_3$. Now based on this selected interest class $C_{kh}^I$ (ref. 122) and said selected manipulation, the determination module 14 is able to determine that the at least one related class $C_{kh}^V$ to be visualized is the class $C_{3,1}^V$ and therefore accessed with the hierarchical structure 21 to the events associated to this class $C_{3,1}^V$. At this point the rendering is updated from panel A to panel B of Figure 9, so that in the graph panel 100 the section of the sunburst chart is folded to show as visualized fraction (i.e., most most external fraction) the fraction 500c and the clusters 510 and 520 of the first graph 101 of panel A have been "merged" so that updated graph 101 (in panel B) shows the one associated cluster 500 of brown dots comprising both dots of previous clusters 510 and 520. In one preferred example, the updating of the rendering is applied on all the scatter plots on which the initial selected interest class $C_{kh}^I$ were visible.

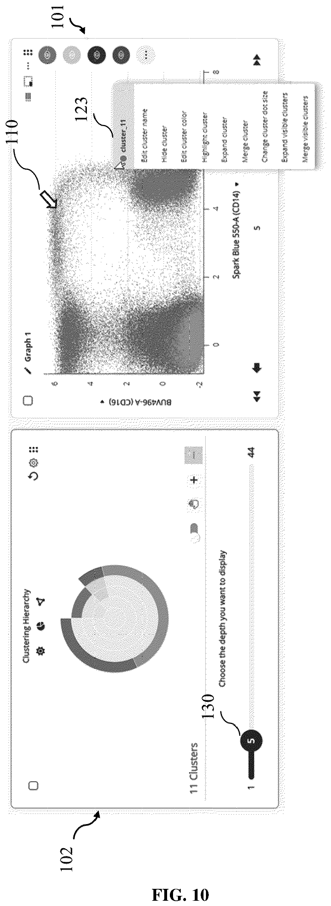

According to another embodiment illustrated in **Figure 10,** the interest class selection component 110 is configured to select on the first graph 101 one cloud of dots and then identify the associated class in the hierarchical structure 21 (as described above). The classes manipulation component 120, which may be open by a right click of the mouse, may be a context menu or a drop-down menu (ref. 123) on which is possible for the user to select the manipulation to apply on the selected interest class $C_{kh}^I$ with a second click.

The graphic panel may further comprise a visual manipulation component for selecting a visual manipulation to apply to one first graph 101. For example, once the interest class is selected (as described in paragraph above), the user may simply decide to remove from the current first graph 101 the cloud of points associated to the selected interest class. As illustrated in Figure 10, other visual manipulation may be available to modify the rendering of the current first graph 101, such as editing the color of the selected dots cloud, change the size of dots for selected dots cloud, highlight cluster. It as to be understood as well, that this visual manipulation component may be configured to select any kind of visual manipulation that may be known by the person skilled in the visualization and analysis of single cell-type data. According to this embodiment, the update rendering 15 is further configured to update the rendering of the graph panel 100 on the base of the selected interest class and the visual manipulation selected by the user.

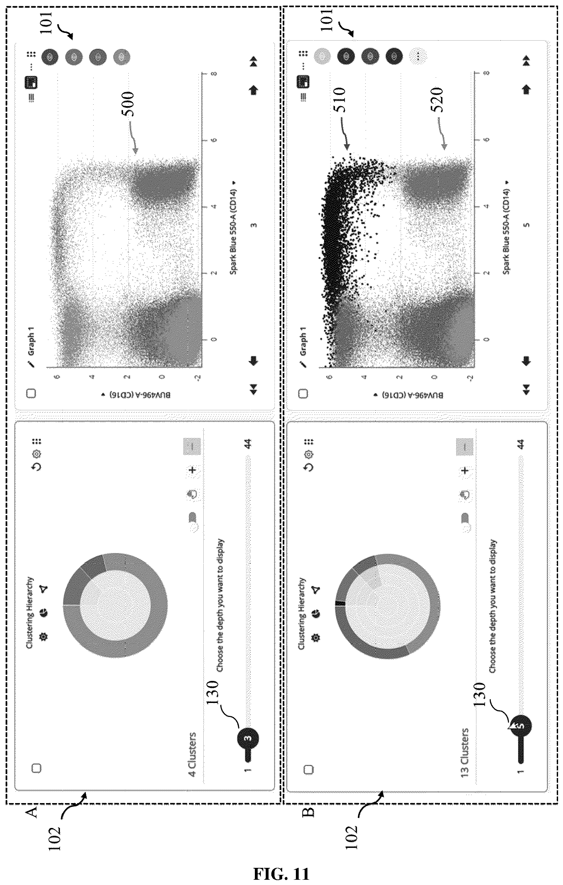

According to one embodiment, that will be illustrated in relation to **Figure 11,** the graph panel 100 further comprises at least one level visualization component 130 (e.g., slider, buttons, arrows, sliders) configured to select one visualization level $L_k^V$. This component 130 may be visualized on the graph panel 100 as a slider, buttons, arrows, multiple sliders, and the like. When the user interacts with this level visualization component 130 via the user interface 50, the module 13 receives a selection of one visualization level $L_k^V$. In the example of Figure 11, the component 130 is visualized as a slider positioned below the second graph 102, and allows by sliding the blue circle cursor to modify the visualization level from 1 ($L^V$) to 44 (i.e., lower level of the hierarchical structure 21 in this example).

In this embodiment, the determining module 14 is configured to use the hierarchical structure 21 to determine at least one visualization classes $C_{kh}^V$ associated to the selected visualization level $L_k^V$ and the events associated to said at least one visualization class $C_{kh}^V$. The updating rendering module 15 is then configured to update the rendering of the graph panel 100 so that both the second graph 102 and first graph 101 are updated to show respectively the first visualization class $C_{kh}^V$ and the events associated to the visualization class $C_{kh}^V$. Advantageously, the level visualization component 130 allows the user to easily modify the granularity of the representation in both the first graph 101 while providing an insightful view on the associated level and associated classes of the hierarchical structure 21 that are visualized on the second graph 102.

In panel A of **Figure 11,** the sunburst graph represents three levels of the hierarchical structure 21 as currently selected by the slider. The lower level (i.e., most external ring) comprises three fractions (i.e., three displeased classes $C_{3h}^V$). The first graph of panel A comprises three corresponding clouds of dots denoted by the same color of the corresponding fraction in the sunburst chart. Then the user slides the cursor on the right so to "expand" the representation, by passing from three to five levels, which provides the visualization illustrated in panel B of Figure 11. In panel B of Figure 11, the sunburst graph 102 comprises five levels, wherein the most external ring is divided into five fractions. The clouds of dots corresponding to these fractions are also rendered in the first graph of panel B.

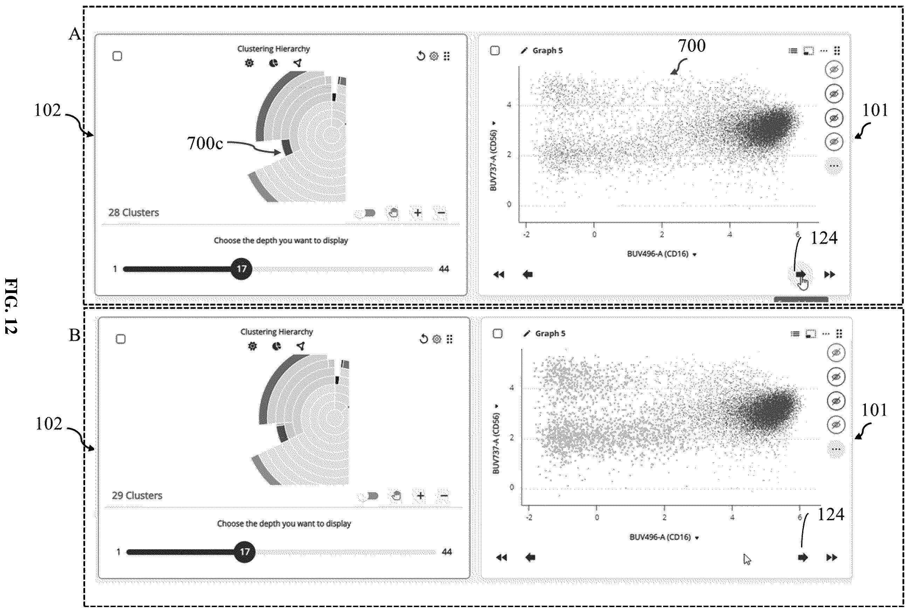

According to one embodiment, the graph panel 100 may comprise further interactive components. In one example illustrated in **Figure 12,** the graph panel 100 comprises an unfold component 124 which is configured to act as both the interest class selection component and classes manipulation component. Indeed, by clicking on the icon associated unfold component 124 the user selects as interest classes $C_{kh}^I$, all the classes currently represented on the associated first graph 101 and select the manipulation of unfold (expand) this(these) interest class(es) $C_{kh}^I$ of one level in the hierarchical structure 21. In Figure 12 panel A, the scatter plot 101 comprises one cloud of points 700 associated with the violet fraction 700c on the sunburst chart. In this scatter plot 101 the user has chosen to mask (i.e., make invisible) all the other clouds of points corresponding to the most external fractions represent in the sunburst 102. Therefore, by clicking on the unfold component 124 the user selected the cloud of points 700 and the corresponding interest class $C_{6h}^I$ (i.e., represented as violet fraction 700c). As already mentioned, the associated manipulation that is applied to this selected class consists in determining the associated one or more classes comprised in the direct upperlevel in the hierarchical structure 21 (i.e., passing from $L_k^V$ to $L_{k+1}^V$). In panel B is represented the updated rendering, wherein the fraction 700c of sunburst chart is not any more the outer fraction for the corresponding section and the scatter plot now distinguishes two populations which are now visible as one yellow and one violet dots cloud.

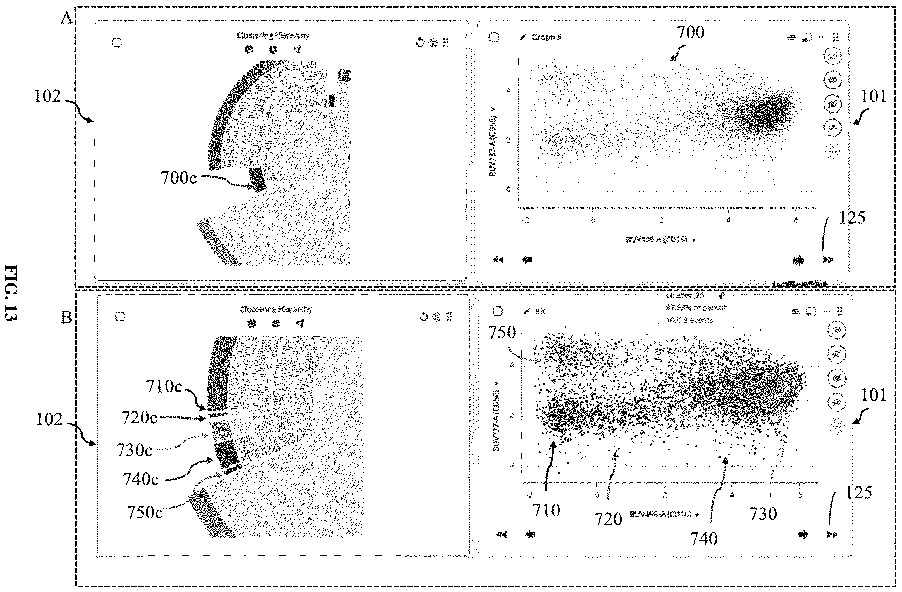

In one other example illustrated in **Figure 13,** the graph panel 100 comprises a "fast unfold" component 125 which is configured to act as both the interest class selection component and classes manipulation component. Indeed, by clicking on the icon associated fast unfold component 125 the user selects as interest classes $C_{kh}^I$, all the classes currently represented on the associated first graph 101 and select the manipulation of unfold (expand) this(these) interest class(es) $C_{kh}^I$ of more than one level in the hierarchical structure 21. In Figure 13 panel A, the scatter plot 101 comprises one cloud of points 700 associated with the violet fraction 700c on the sunburst chart. As before in figure 12 panel A, in this scatter plot 101 the user has chosen to mask all the other clouds of points. Therefore, by clicking on the fast unfold component 125 the user selected the one cloud of points 700 and the corresponding interest class $C_{6h}^I$ (i.e., represented as violet fraction 700c). The associated manipulation illustrated in Figure 13 is that of determining the associated one or more classes comprised in the hierarchical structure 21 three level higher (i.e., passing from $L_k^V$ to $L_{k+3}^V$). So that when the user clicks on fast unfold component 125 the results obtained is illustrated in panel B. The fraction 700c of sunburst chart is not any more the outer fraction for the corresponding section, but five fractions (710c, 720c, 730c, 740c and 750c) comprised in both the 9th level of the sunburst chart 102 and the same section of fraction 700c. Simultaneously, the scatter plot is updated to show the five corresponding dots clouds (710, 720, 730, 740 and 750).

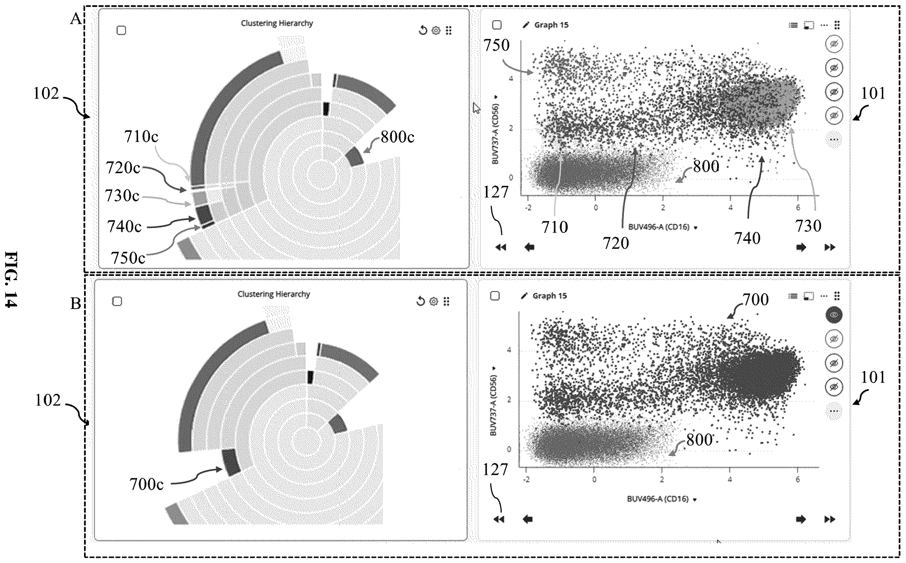

In one other example illustrated in **Figure 14,** the graph panel 100 comprises a "fast fold" component 127 which is configured to act as both the interest class selection component and classes manipulation component. Indeed, by clicking on the icon associated fast fold component 127 the user selects as interest classes $C_{kh}^I$, all the classes currently represented on the associated first graph 101 and select the manipulation of fold (merge) this(these) interest class(es) $C_{kh}^I$ of more than one level in the hierarchical structure 21. In Figure 14 panel A, the scatter plot shows the six dot clouds (710, 720, 730, 740, 750 and 760) chosen by the user for visualization (e.g., the dots clouds associated to the red, gray and brown fractions are masked). Therefore, by clicking on the fast fold component 127 the user selected the six clouds of dots (710, 720, 730, 740, 750 and 800) and the corresponding interest classes (i.e., corresponding to the fractions 710c, 720c, 730c, 740c, 750c and 800c of the sunburst chart) in level $L_9$. The associated manipulation illustrated in Figure 14 is that of determining the associated one or more classes comprised in the hierarchical structure 21 three level lower than the level of the interest classes (i.e., passing from

$$L_k^V \quad \text{to} \quad L_{k-3}^V$$

). As easily illustrated in the sunburst chart, the fractions 710c, 720c, 730c, 740c and 750c and the fraction 800c do not belong to the same section till the lower level $L_2$. As consequence, when applying the manipulation consisting in searching for common parents in the hierarchical structure 21 at a level that is three level lower than the selected interest classes, only the fractions 710c, 720c, 730c, 740c and 750c are folded (i.e. disappeared) to leave the place to fraction 700c. The fraction 800c is unchanged as it lies in a lower level. The corresponding five dot clouds are therefore merged in the scatter plot 101, while the cloud 800 remains unmodified. Advantageously, using the hierarchical structure 21 for merging population of events allow to only group together dot clouds belonging to a commune parent class, leaving untouched the dot clouds that have not direct connection at the chosen level of the hierarchical structure 21.

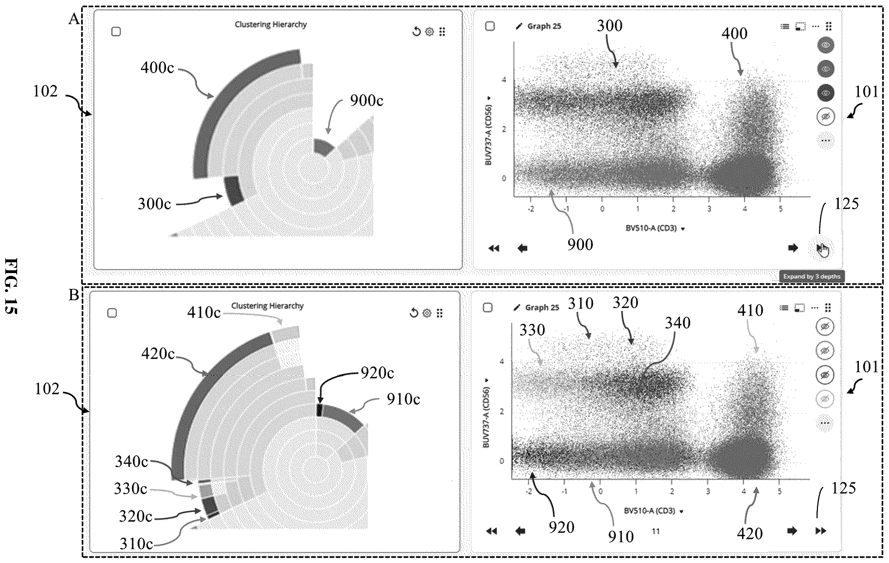

**Figure 15** provides another example of use of the "fast unfold" component 125. In Figure 15 panel A, the scatter plot 101 comprises three clouds of dots (300 violet, 400 red and 900 brown), each one associated with the corresponding fraction on the sunburst chart (300c violet, 400c red and 900c brown). As before in figure 12 panel A, in this scatter plot 101 the user has chosen to mask all the other clouds of dots (i.e., the cloud of dots associated to the grey fraction in the sun burst). Now if the user wants to explore more in detail the visualised populations, in other words augment the granularity of the representation, he/she may click on the component 125 to "fast unfold" the hierarchical structure 21. Indeed, by clicking on the fast unfold component 125 the user selected the three clouds of dots 300, 400 and 900 (therefore selecting the corresponding interest classes represented as the 300c violet fraction, 400c red fraction and 900c brown fraction), plus the manipulation consisting in changing the visualisation level by reducing it of three levels (i.e., passing from $L_k^V$ to

$L_{k-3}^V$ ).

Panel B shows the updated rendering based on these three selected interest classes and said at least one selected manipulation. In this example, it is interesting to notice that each branch of the hierarchical structure 21 associated to the three selected interest classes unfold of three levels independently. Indeed, the fraction 300c of sunburst chart is not any more the outer fraction for the corresponding section, but four fractions (310c, 320c, 330c and 340c) comprised in both the 8th level of the sunburst chart 102 and the same section of previous fraction 300c. In the same way, the fraction 900c is replaced by the two external fraction 910c (brown) and 920c (blue) in the fourth level and same section of previous fraction 900c, while the fraction 400c is replaced by the two external fraction 410c (green) and 420c (red) in the 10th level and same section of previous fraction 400c of the sunburst. Simultaneously, the scatter plot is updated to show the corresponding dots clouds so that in panel B the initial dot cloud 400 is now segmented in two dot clouds (410 and 420) and the initial dot cloud 900 is now segmented in two dot clouds (910 and 920).

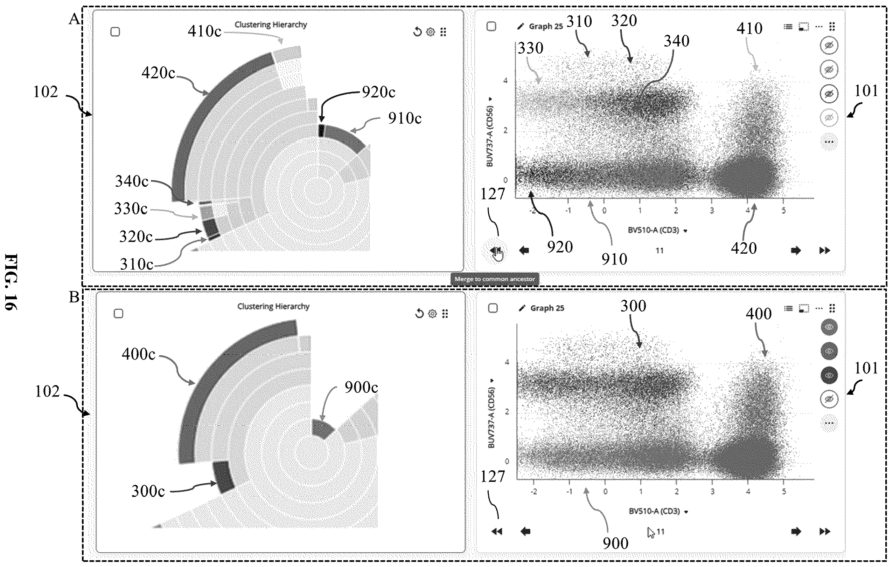

**Figure 16** provides another example of use of the "fast fold" component 127 for the same example illustrated in Figure 15, showing that the present embodiment advantageously allows the user to explore easily and intuitively the hierarchical structure both ways with a simple click.

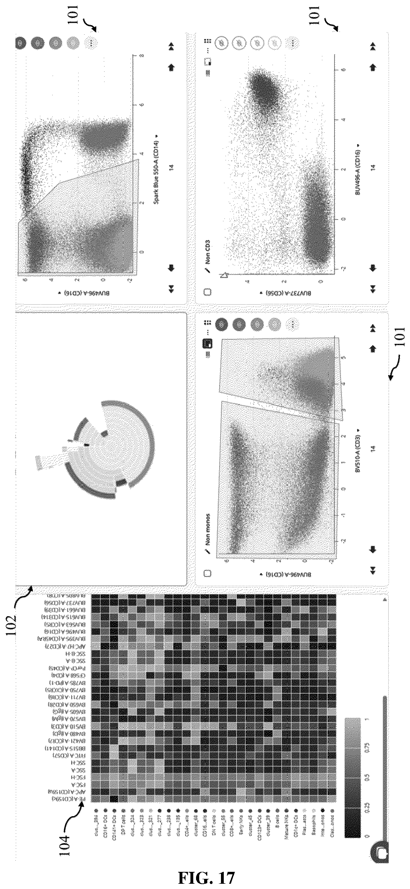

As previously disclosed, other graphs than the second graph 102 and first graph 101 may be displayed (rendered) at the same time in the graph panel 100. The graph panel 100 may further comprises quantitative values information graph 104 configured to provide a representation of the fluorescent information. One example of fluorescent information graph 104 is a color-coded heatmaps graph as illustrated in Figure 17. Indeed, intensities for each parameter can be displayed as color-coded heatmaps, where events are represented by a colored pixel corresponding to its intensity. Heatmaps allow for the visualization of patterns and heterogeneity in parameter intensity across different cell populations or experimental conditions. This visual representation of graph 104 helps researchers to interpret data, identify cell populations of interest, and gain insights into cellular heterogeneity and functional characteristics.

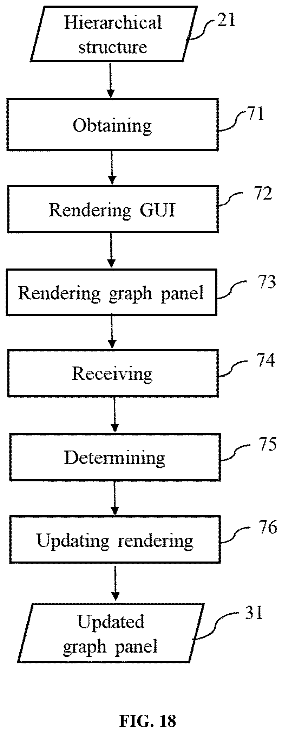

In its automatic actions, the device 1 may for example execute the following process (**Figure 18**):

- obtaining at least one hierarchical structure 21 representative of a plurality of classes of biological objects $C_{kh}$ and mutual relationships between said plurality of classes $C_{kh}$ of biological objects (step 41),

- rendering a graphical user interface on the visual display device 60 and rendering, on the graphical user interface, the graph panel 100 with the at least one first graph 101, the interest class selection component 110 and the classes manipulation component 120 (step 42),

- receiving at least one selected interest class $C_{kh}^I$ and at least one selected manipulation to apply (step 43),

- based on said at least one selected interest class ( $C_{kh}^I$ ) and said at least one selected manipulation, determining at least one related class ( $C_{kh}^V$ ) and associated events to be visualized (step 44),
- updating the rendering of said graph panel (100) so that the at least one first graph (101) shows at least the associated events associated to said at least one related class $C_{kh}^V$ (step 45).

## Claims

1. A computer-implemented method for clustering and visualization of single cell-type data associated with a plurality of biological objects selected from cells, cellular origin vesicles, microorganisms, acellular microorganisms, particles, and/or biofunctionalized materials; said single cell-type data comprising N events, each associated with a biological object, each cell event being defined by at least two biological object parameters measured for the corresponding biological object, said method comprising:

   - obtaining at least one hierarchical structure (21) representative of a plurality of classes of biological objects ($C_{kh}$) and mutual relationships between said plurality of classes ($C_{kh}$) of biological objects; wherein said at least one hierarchical structure, obtained applying a hierarchical clustering method on said single cell-type data, comprises a plurality of classes ($C_{kh}$) and associated levels ($L_k$), wherein each class ($C_{kh}$) is associate to at least one cell event and is representative of at least one biological object and each level ($L_k$) is representative of a different degree of similarity among said classes ($C_{kh}$);
   - rendering a graphical user interface on a visual display device (60) of a system (1);
   - rendering, on the graphical user interface, a graph panel (100) comprising:

      o at least one first graph (101) being a bi- or tri-dimensional scatter plot graphically showing at least a portion of cell events with respect to two biological object parameters, so that said portion of events is represented by a cloud of dots in a 2- or 3-dimensional plot;
      o an interest class selection component (110) for selecting at least one interest class ( $C_{kh}^I$ ) of the at least one hierarchical structure (21);
      o a classes manipulation component (120) for selecting a manipulation to apply to said at least one selected interest class ( $C_{kh}^I$ ), said manipulation comprising using the hierarchical structure (21) for:

         ▪ obtaining at least one relationship between said at least one selected interest class ( $C_{kh}^I$ ) and at least one among the remaining plurality of classes ($C_{kh}$), or
         ▪ obtaining the selected interest class ( $C_{kh}^I$ );

   - receiving at least one selected interest class ( $C_{kh}^I$ ) and at least one selected manipulation to apply;
   - based on said at least one selected interest class ( $C_{kh}^I$ ) and said at least one selected manipulation, determining at least one related class ( $C_{kh}^V$ ) and associated cell events to be visualized;
   - updating the rendering of said graph panel (100) so that the at least one first graph (101) shows at least the associated cell events associated to said at least one related class ( $C_{kh}^V$ ).

2. The computer-implemented method according to claim **1,** wherein the manipulation consists in determining the one or more upper level classes associated to the selected at least one interest class ( $C_{kh}^I$ ) in the hierarchical structure (21).

3. The computer-implemented method according to claim **1,** wherein the manipulation consists in determining the one or more lower level classes associated to the at least one interest class ( $C_{kh}^I$ ) in the hierarchical structure (21).

4. The computer-implemented method according to claim **2,** wherein the manipulation consists in receiving at least two selected interest class ( $C_{kh}^I$ ) and merging them.

5. The computer-implemented method according to any one of claims **1** to **4,** wherein the graph panel further comprises a second graph (102), obtained from the received hierarchical structure, graphically showing said mutual relationships between said

plurality of classes ($C_{kh}$) of biological objects and said associated levels ($L_k$) and wherein updating the rendering of said graph panel further comprises updating the rendering of said second graph (102) so as to graphically shows the determined at least one related class ( $C_{kh}^{V}$ ).

6. The computer-implemented method according to claim **5,** wherein said second graph (102) is a sunburst chart comprising concentric rings, each ring representing a level ($L_k$) and having a diameter increasing as it represents a lower hierarchical level, and wherein the classes ($C_{kh}$) at a level are fractions of the corresponding ring at that level ($L_k$).

7. The computer-implemented method according to claim **5,** wherein said second graph (102) is a dendrogram representation.

8. The computer-implemented method according to any one of claims **1** to **7,** wherein said interest class selection component (110) for selecting at least one interest class ( $C_{kh}^{I}$ ) is configured to:

   - delineate a region of interest in the at least one first graph (101) comprising a plurality dots, said plurality of dots being associated to at least one class ($C_{kh}$);
   - calculating a centroid for each sub-ensemble of said plurality of dots associated to one same class ($C_{kh}$) comprised in said region of interest;
   - using each calculated centroid, identifying in the hierarchical structure (21) the at least one interest class ( $C_{kh}^{I}$ ) to which are associated the dots comprised in said region of interest.

9. The computer-implemented method according to any one of claims **5** to **7,** wherein said interest class selection component (110) is configured to select the at least one interest class ( $C_{kh}^{I}$ ) from the second graph (102) by means of a user interface (50).

10. The computer-implemented method according to any one of claims **5** to **9,** wherein the graph panel (100) further comprises at least one level visualization component (130) configured to select a visualization level ( $L_{k}^{V}$ ) and, when a visualization level ( $L_{k}^{V}$ ) is received, the method is further configured to:

    - determine, using the hierarchical structure (21), at least one visualization classes ($C_{kh}$)

associated to said visualization level ( $L_{k}^{V}$ ) and the cell events associated to said at least one visualization class;
- update the graph panel rendering so that the second graph (102) shows at least one visualization class ($C_{kh}$) and the first graph (101) shows the determined cell events associated to said at least one visualization class ($C_{kh}$).

11. A system (1) for clustering and visualization of single cell-type data, the system comprising:

    - a visual display device (60);
    - a user interface (50), and
    - at least one computer processor (10) coupled to the visual display device (60) and the user interface (50) programmed to perform the method according to any one of claims **1** to **10.**

12. A non-transitory computer-readable medium having encoded thereon computer-executable instructions that, when executed on at least one computer processor (10) according to claim **11,** perform the computer-implemented method according to any one of claims **1** to **10.**

**FIG. 1**

**FIG. 2**

**FIG. 3**

FIG. 4

FIG. 5

FIG. 6

FIG. 7

FIG. 8

**FIG. 9**

FIG. 10

28

EP 4 645 321 A1

FIG. 11

**FIG. 12**

FIG. 13

FIG. 14

**FIG. 15**

**FIG. 16**

**FIG. 17**

Hierarchical structure /21

↓

Obtaining /71

↓

Rendering GUI /72

↓

Rendering graph panel /73

↓

Receiving /74

↓

Determining /75

↓

Updating rendering /76

↓

Updated graph panel /31

**FIG. 18**

# EUROPEAN SEARCH REPORT

Europäisches
Patentamt
European
Patent Office
Office européen
des brevets

**Application Number**

EP 24 30 5710

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/094625 A1 (METAFORA BIOSYSTEMS [FR] ET AL.) 1 June 2023 (2023-06-01) * paragraphs [0072] - [0076], [0078] - [0080], [0083], [0213] - [0216]; claim 1; figures 1,4,8,9,10 * ----- | 1-12 | INV. G16B40/30 G16B45/00 |
| A | US 10 289 802 B2 (QIU PENG [US]; GENTLES ANDREW J [US] ET AL.) 14 May 2019 (2019-05-14) * the whole document * ----- | 1-12 | |
| A | PHILIPPE HAUCHAMPS ET AL: "CytoPipeline and CytoPipelineGUI: a Bioconductor R package suite for building and visualizing automated pre-processing pipelines for flow cytometry data", BMC BIOINFORMATICS, BIOMED CENTRAL LTD, LONDON, UK, vol. 25, no. 1, 20 February 2024 (2024-02-20), pages 1-22, XP021332541, DOI: 10.1186/S12859-024-05691-Z * the whole document * ----- | 1-12 | |
| A | KAREL FISER ET AL: "Detection and monitoring of normal and leukemic cell populations with hierarchical clustering of flow cytometry data", CYTOMETRY A, WILEY-LISS, HOBOKEN, USA, no. 1, 11 October 2011 (2011-10-11), pages 25-34, XP072332565, ISSN: 1552-4922, DOI: 10.1002/CYTO.A.21148 * the whole document * ----- | 1-12 | **TECHNICAL FIELDS SEARCHED (IPC)** G16B |

The present search report has been drawn up for all claims

| Place of search | Date of completion of the search | Examiner |
|---|---|---|
| Munich | 16 October 2024 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P4C01)

## ANNEX TO THE EUROPEAN SEARCH REPORT
## ON EUROPEAN PATENT APPLICATION NO.

EP 24 30 5710

This annex lists the patent family members relating to the patent documents cited in the above-mentioned European search report.
The members are as contained in the European Patent Office EDP file on
The European Patent Office is in no way liable for these particulars which are merely given for the purpose of information.

16-10-2024

| Patent document cited in search report | Publication date | Patent family member(s) | Publication date |
|---|---|---|---|
| WO 2023094625 A1 | 01-06-2023 | EP 4437513 A1 | 02-10-2024 |
|  |  | WO 2023094625 A1 | 01-06-2023 |
| US 10289802 B2 | 14-05-2019 | NONE |  |

EPO FORM P0459

For more details about this annex : see Official Journal of the European Patent Office, No. 12/82

**REFERENCES CITED IN THE DESCRIPTION**

**Patent documents cited in the description**

- WO 2023094625 A

- WO 20230946625 A